# EUROPÄISCHE PATENTSCHRIFT

(11) **EP 1 667 956 B1**
(45) Veröffentlichungstag und Bekanntmachung des Hinweises auf die Patenterteilung: **13.06.2007**
(21) Anmeldenummer: 04765603.8
(22) Anmeldetag: 24.09.2004
(51) Int. Cl.: C07C 209/86, C07C 209/90, C07C 211/04, C07C 209/16

(54) **VERFAHREN ZUR VERMEIDUNG VON KORROSION**
METHOD FOR AVOIDING CORROSION
PROCEDE POUR EVITER LA CORROSION

(30) Priorität: 24.09.2003 DE 10344282
(43) Veröffentlichungstag der Anmeldung: 14.06.2006
(73) Patentinhaber: BASF Aktiengesellschaft, 67056 Ludwigshafen (DE)
(72) Erfinder: REUTEMANN, Werner, 67240 Bobenheim-Roxheim (DE); WEBER, Theodor, 67063 Ludwigshafen (DE); ROSS, Karl-Heinz, 67269 Grünstadt (DE); JULIUS, Manfred, 67117 Limburgerhof (DE)
(74) Vertreter: Isenbruck, Günter
(86) Internationale Anmeldenummer: PCT/EP2004/010766
(87) Internationale Veröffentlichungsnummer: WO 2005/030697

(56) Entgegenhaltungen:
- EP-A- 0 037 695
- EP-A- 1 312 599
- PATENT ABSTRACTS OF JAPAN Bd. 0061, Nr. 97 (C-128), 6. Oktober 1982 (1982-10-06) & JP 57 108041 A (MITSUBISHI GAS CHEM CO INC), 5. Juli 1982 (1982-07-05)
- CHEMICAL ABSTRACTS, Bd. 55, Nr. 24, 1961, Columbus, Ohio, US; abstract no.: 244489i, A.M. SUKHOTIN ET AL.: "Corrosion of installations for the production of methylamines." Seite 24490 Spalte 1 XP002312527 & TRUDY GOSUDARST. INST. PRIKLAD. KHIM., Bd. 44, 1960, Seiten 28-40,

## Beschreibung

Die Erfindung betrifft ein Verfahren zur Vermeidung von Korrosion bei der Abtrennung von Methylaminen aus einem bei der Herstellung von Methylaminen erhaltenen Produktstrom.

Bei der Reaktion von Methanol und Ammoniak in Gegenwart eines heterogenen Katalysators auf der Basis von Aluminiumoxid werden Monomethylamin, Dimethylamin und Trimethylamin gebildet. Die Reaktion zur Bildung der Methylamine ist exotherm und erfolgt bei einer Temperatur von 390 bis 430°C. Da es sich bei den Reaktionen zur Bildung von Methylaminen um Gleichgewichtsreaktionen handelt, sind neben den Methylaminen Ammoniak und Methanol im Produktstrom enthalten.

Nach der Reaktion wird der Produktgasstrom einer Destillationsanlage zugeführt. In der Destillationsanlage wird der Produktgasstrom in die einzelnen Komponenten aufgetrennt. In den zur Zeit verwendeten Destillationsanlagen wird in einer ersten Kolonne Ammoniak, welches mit Trimethylamin ein Azeotrop bildet, wodurch auch ein Teil des Trimethylamins mit abdestilliert wird, in einer zweiten Kolonne Trimethylamin und in einer dritten Kolonne Wasser abgetrennt, das in der Regel bei der Reaktion nicht umgesetztes Methanol enthält. Über den Kopf der dritten Kolonne wird ein Monomethylamin und Dimethylamin enthaltender Gasstrom abgezogen und einer vierten Kolonne zugeführt. In der vierten Kolonne wird der Gasstrom in Monomethylamin und Dimethylamin aufgetrennt. Zur Abtrennung des Methanols aus dem methanolhaltigen Wasser der dritten Kolonne kann der dritten Kolonne eine weitere Kolonne nachgeschaltet sein. Das in der weiteren Kolonne erhaltene Methanol wird wie der in der ersten Kolonne abgetrennte Ammoniak erneut der Methylaminsynthese zugeführt.

Aufgrund der Aggressivität der Komponenten im Produktstrom korrodieren die vorzugsweise aus C-Stahl gefertigten Destillationskolonnen. Zur Vermeidung der Korrosion ist es bekannt, in Alkylaminanlagen dem Zulauf der ersten Kolonne Alkalihydroxid zur Korrosionsverhinderung zuzugeben. Bei der Zugabe von Alkalihydroxid in den Zulauf der ersten Kolonne der Methylaminanlage treten jedoch bereits nach kurzer Zeit Verstopfungen auf den Böden der ersten Kolonne auf.

Verfahren zur destillativen Abtrennung von Methylaminen aus einem Methylamin enthaltenden Produktgasstrom sind zum Beispiel aus EP-A 0 037 695, aus EP-A 1 312 599 oder aus JP-A 57108041 bekannt. In Chemical Abstracts, Bd. 55, Nr. 24, 1961, Columbus, Ohio, US, Abstract Nr. 244489i ist die Korrosion von Vorrichtungen zur Produktion von Methylaminen beschrieben.

Es war Aufgabe der Erfindung, ein Verfahren zur Vermeidung von Korrosion in den Kolonnen der Destillationsanlage bei der Methylaminherstellung bereitzustellen.

Die erfindungsgemäße Lösung besteht darin, in den Zulauf zur dritten Kolonne Alkalihydroxid zuzugeben. Bei der Zugabe des Alkalihydroxides in den Zulauf zur dritten Kolonne zeigt sich, dass es auch in der ersten und zweiten Kolonne keine Korrosion gibt, obwohl dort aggressive Medien im Gasstrom enthalten sind. Darüber hinaus wird durch die erfindungsgemäße Lösung die Ausbildung von Verstopfungen in der ersten und zweiten Kolonne vermieden.

Zur Gewinnung von Monomethylamin, Dimethylamin und Trimethylamin aus dem bei der Reaktion aus Ammoniak und Methanol gewonnenen Produktgasstrom wird der Produktgasstrom einer Destillationsanlage zugeführt. Der Produktgasstrom wird in einem Seitenzulauf einer ersten Destillationskolonne zugeführt. In der ersten Kolonne wird Ammoniak durch Destillation abgetrennt. Die Destillation erfolgt bei einem Druck von vorzugsweise 15 bis 20 bar und insbesondere bei einem Druck von 15 bis 18 bar. Der als Azeotrop mit Trimethylamin vorliegende abgetrennte Ammoniak wird über den Kopf der ersten Kolonne abgezogen und vorzugsweise erneut der Methylaminherstellung zugeführt. Die übrigen Bestandteile des Produktgasstromes bilden den Sumpf und werden aus der Kolonne abgezogen und einer zweiten Kolonne zugeführt. Der Zulauf der zweiten Kolonne ist ebenfalls als Seitenzulauf ausgebildet. In der zweiten Kolonne wird durch eine Extraktivdestillation unter Zugabe von Wasser Trimethylamin abgetrennt. Das Trimethylamin wird über den Kopf der zweiten Kolonne abgezogen. Die den Sumpf bildenden restlichen Komponenten des Produktgasstromes werden in einem Seitenzulauf einer dritten Kolonne zugeführt. Über den Sumpf der dritten Kolonne werden das zur Extraktivdestillation in der zweiten Kolonne eingesetzte Wasser und das bei der Reaktion gebildete Wasser, sowie nicht umgesetztes Methanol abgezogen. Über den Kopf der dritten Kolonne wird ein Gemisch aus Monomethylamin und Dimethylamin abgezogen. Die Destillation in der dritten Kolonne erfolgt vorzugsweise bei einem Druck von 7 bis 15 bar und insbesondere bei einem Druck von 8 bis 12 bar. Das über den Kopf der dritten Kolonne abgezogene Gemisch aus Monomethylamin und Dimethylamin wird in einem Seitenzulauf einer vierten Kolonne zugeführt. In der vierten Kolonne wird der Strom aus Monomethylamin und Dimethylamin destillativ bei einem Druck von vorzugsweise 6 bis 10 und insbesondere bei einem Druck von 7 bis 9 bar aufgetrennt. Im Sumpf der vierten Kolonne fällt Dimethylamin an und über den Kopf der vierten Kolonne wird Monomethylamin abgezogen.

Zur Abtrennung des Methanols aus dem bei der Destillation in der dritten Kolonne anfallenden Wasser kann eine fünfte Kolonne eingesetzt werden, der aus einem Seitenabzug der dritten Kolonne das methanolhaltige Wasser zugeführt wird. In der fünften Kolonne wird durch Destillation das Methanol abgetrennt. Das Methanol wird über den Kopf der fünften Kolonne abgezogen und erneut der Reaktion zugeführt. Das als Sumpf der fünften Kolonne anfallende von Methanol gereinigte Wasser wird zurück in die dritte Kolonne geführt.

Bei der erfindungsgemäßen Lösung zur Vermeidung von Korrosion in der Destillationsanlage wird in den Zulauf zur dritten Kolonne Alkalihydroxid gegeben. Als Alkalihydroxid im Sinne der Erfindung eignen sich insbesondere Natriumhydroxid und Kaliumhydroxid. Die Menge an Alkalihydroxid muss so bemessen sein, dass sich im Sumpf der dritten Kolonne nicht umgesetztes Alkalihydroxid befindet.

Die Beheizung der Kolonnen erfolgt vorzugsweise durch Dampf mit einem Druck von vorzugsweise 10 bis 20 bar und insbesondere einem Druck im Bereich von 12 bis 17 bar im Sumpf der Kolonnen.

Die zur Destillation eingesetzten Kolonnen sind vorzugsweise Bodenkolonnen. Als Kolonnenböden eignen sich alle dem Fachmann bekannten Bauarten. Neben Bodenkolonnen können aber auch Packungskolonnen oder Füllkörperkolonnen eingesetzt werden. Hierbei kann jede beliebige dem Fachmann bekannte Füllkörpergeometrie eingesetzt werden.

Der Transport des Produktstromes durch die Kolonnenkaskade erfolgt vorzugsweise aufgrund der Druckdifferenz zwischen den einzelnen Kolonnen.

Im Produktgasstrom sind neben Monomethylamin, Dimethylamin und Trimethylamin bei der Reaktion nicht umgesetztes Methanol und Ammoniak sowie als Reaktionsnebenprodukt gebildetes Wasser und weitere Nebenprodukte enthalten. Von diesen Nebenprodukten wirken insbesondere Kohlendioxid, Ammoniumcarbamat und Ameisensäure korrosiv gegenüber Eisen. Durch Zugabe einer Base zur Neutralisation der Säuren und zur Bildung einer basischen Umgebung kann die Korrosion des Eisens vermindert beziehungsweise unterbunden werden.

Als Base zur Korrosionsvermeidung wird vorzugsweise Alkalihydroxid zugegeben, insbesondere Natriumhydroxid oder Kaliumhydroxid. Das Alkalihydroxid kann dabei in fester Form als Salz oder bevorzugt als wässrige Lösung zugegeben werden. Bei Einsatz einer wässrigen Alkalihydroxidlösung weist diese vorzugsweise eine Konzentration von 25 % auf.

Überraschenderweise zeigt sich bei Zugabe des Natriumhydroxides in den Zulauf zur dritten Kolonne, dass in der Destillationsanlage keine Korrosion auftritt und auch keine Böden verstopfen.

Die Menge des zudosierten Alkalihydroxides ist so zu bemessen, dass im Sumpf der dritten Kolonne noch Alkalihydroxid enthalten ist.

Neben der Zugabe in den Zulauf zur dritten Kolonne kann das Alkalihydroxid auch in den Sumpf oder in den Abtriebsteil der zweiten Kolonne direkt zugegeben werden.

Im Folgenden wird die Erfindung anhand einer Zeichnung und eines Beispiels näher erläutert.

Es zeigt:

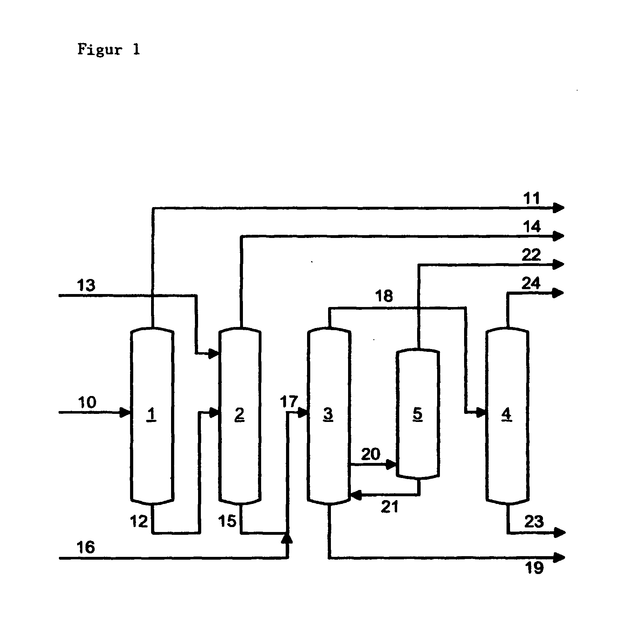

- Figur 1: eine erfindungsgemäß ausgebildete Destillationsanlage zur Methylamindestillation.

Eine erfindungsgemäß ausgebildete Destillationsanlage zur Methylamindestillation umfasst gemäß der Figur fünf Kolonnen. Ein bei der Reaktion von Ammoniak und Methanol zu Methylaminen anfallender Produktgasstrom 10 wird einer ersten Kolonne 1 über einen Seitenzulauf zugeführt. In der ersten Kolonne 1 wird Ammoniak, welcher als Azeotrop mit Trimethylamin vorliegt, aus dem Produktstrom durch Destillation abgetrennt. Über den Kopf der ersten Kolonne 1 wird Ammoniak 11 abgezogen und erneut der Methylaminsynthese zugeführt. Die restlichen Komponenten des Produktstromes 10 fallen als Sumpf 12 der ersten Kolonne 1 an. Der Sumpf 12 der ersten Kolonne 1 wird über einen Seitenzulauf einer zweiten Kolonne 2 zugeführt. In der zweiten Kolonne 2 wird durch eine Extraktivdestillation Trimethylamin aus dem Sumpf 12 der ersten Kolonne 1 abgetrennt. Für die Extraktivdestillation wird in die zweite Kolonne 2 Wasser 13 über einen zweiten Seitenzulauf zugegeben. Der zweite Seitenzulauf liegt oberhalb des Zulaufs für den Sumpf 12 der ersten Kolonne 1. Über den Kopf der zweiten Kolonne 2 wird Trimethylamin 14 abgezogen. Die restlichen Komponenten sammeln sich im Sumpf 15 der zweiten Kolonne 2. Der Sumpf 15 der zweiten Kolonne 2 wird als Zulauf 17 einer dritten Kolonne 3 zugeführt. In den Zulauf 17 wird Lauge 16 zugegeben. Neben der Zugabe der Lauge 16 in den Zulauf 17 zur dritten Kolonne 3 kann die Lauge 16 auch in den Sumpf 15 oder in den Abtriebsteil der zweiten Kolonne 2 zugeführt werden. Die Lauge 16 ist vorzugsweise ein Alkalihydroxid und insbesondere Natriumhydroxid oder Kaliumhydroxid in wässriger Lösung.

In der dritten Kolonne 3 wird durch Destillation aus dem Sumpf 15 der zweiten Kolonne 2 Monomethylamin und Dimethylamin abgetrennt. Das Monomethylamin und Dimethylamin wird als Kopfstrom 18 über den Kopf der dritten Kolonne 3 abgezogen und einer vierten Kolonne 4 zugeführt. Im Sumpf der dritten Kolonne 3 sind Wasser, Methanol und weitere Reaktionsnebenprodukte enthalten. Zur Abtrennung des Methanols aus dem Sumpf der dritten Kolonne 3 kann der dritten Kolonne 3 eine fünfte Kolonne 5 nachgeschaltet werden. Der fünften Kolonne 5 wird über einen Zulauf 20 methanolhaltiges Wasser aus der dritten Kolonne 3 zugeführt. Durch Destillation wird in der fünften Kolonne 5 Methanol aus dem Wasser abgetrennt. Das von Methanol gereinigte Wasser wird über eine Rücklauf 21 wieder in die dritte Kolonne 3 zurückgeführt. Über den Kopf der fünften Kolonne 5 wird das abgetrennte Methanol 22 abgezogen und erneut der Methylaminsynthese zugeführt. Aus dem Sumpf der dritten Kolonne 3 wird von Methanol gereinigtes Abwasser 19 abgezogen.

In der vierten Kolonne 4 wird der Kopfstrom 18 der dritten Kolonne 3, der insbesondere Monomethylamin und Dimethylamin enthält, in Monomethylamin und Dimethylamin aufgetrennt. Über den Kopf der vierten Kolonne 4 wird Monomethylamin 24 abgezogen. Aus dem Sumpf der vierten Kolonne 4 wird anfallendes Dimethylamin 23 abgezogen.

### Beispiel

In einer Destillationsanlage wird ein bei der Synthese von Methylamin anfallender Produktstrom aufgetrennt. Die Beheizung der Kolonnen erfolgt über Wasserdampf mit einem Druck von 16 bar. Aus dem Produktstrom wird in einer ersten Kolonne bei einem Druck von 16,5 bar Ammoniak, der als Azeotrop mit Trimethylamin vorliegt, abgetrennt. Der restliche Produktstrom wird einer zweiten Kolonne zugeführt. In der zweiten Kolonne wird bei einem Druck von 14 bar bei einer Sumpftemperatur von 160°C und einer Kopftemperatur von 103°C Trimethylamin abgetrennt und über den Kopf der zweiten Kolonne abgezogen. Der Sumpf der zweiten Kolonne wird einer dritten Kolonne zugeführt, wobei in den Zulauf Natriumhydroxidlösung zugegeben wird. In der dritten Kolonne wird bei einem Druck von 8,3 bar mit einer Sumpftemperatur von 178°C und einer Kopftemperatur von 68°C über den Kopf Dimethylamin und Monomethylamin abgezogen. Das Monomethylamin und Dimethylamin wird in einer vierten Kolonne bei einem Druck von 7,5 bar mit einer Sumpftemperatur von 74°C und einer Kopftemperatur von 53°C aufgetrennt. Über den Kopf der vierten Kolonne wird Monomethylamin abgezogen und über den Sumpf der vierten Kolonne Dimethylamin. Das als Sumpf der dritten Kolonne anfallende methanolhaltige Wasser wird über einen Seitenabzug am Fuß der dritten Kolonne dampfförmig einer fünften Kolonne zugeführt, die bei einem Druck von 8,3 bar mit einer Sumpftemperatur von 170°C und einer Kopftemperatur von 165°C arbeitet. Über den Kopf der fünften Kolonne wird Methanol abgezogen. Das von Methanol gereinigte Wasser wird aus dem Sumpf der fünften Kolonne wieder der dritten Kolonne zugeführt. Die eingesetzte Natriumhydroxidlösung hat eine Konzentration von 25 % NaOH. Bei der so durchgeführten Methylamindestillation zeigt sich keine Korrosion in den Kolonnen.

### Bezugszeichenliste

- 1: erste Kolonne
- 2: zweite Kolonne
- 3: dritte Kolonne
- 4: vierte Kolonne
- 5: fünfte Kolonne
- 10: Produktstrom
- 11: Ammoniak
- 12: Sumpf der ersten Kolonne 1
- 13: Wasser
- 14: Trimethylamin
- 15: Sumpf der zweiten Kolonne 2
- 16: Lauge
- 17: Zulauf zur dritten Kolonne 3
- 18: Kopfstrom der dritten Kolonne 3
- 19: Abwasser
- 20: Zulauf zur fünften Kolonne 5
- 21: Rücklauf der fünften Kolonne 5
- 22: Methanol
- 23: Dimethylamin
- 24: Monomethylamin

## Patentansprüche

1. Verfahren zur Vermeidung von Korrosion bei der Abtrennung von Methylaminen aus einem bei der Herstellung von Methylaminen durch Gasphasenreaktion aus Methanol und Ammoniak anfallenden, Monomethylamin, Dimethylamin, Trimethylamin, Ammoniak und Methanol als Komponenten enthaltenden Produktstrom (10), wobei in einer ersten Kolonne (1) Ammoniak (11) durch reine Destillation abgetrennt wird, die als Sumpf (12) anfallenden restlichen Komponenten des Produktstromes einer zweiten Kolonne (2) zugeführt werden, in der zweiten Kolonne (2) durch eine Extraktivdestillation unter Zufuhr von Wasser Trimethylamin (14) abgetrennt wird, die als Sumpf (15) der zweiten Kolonne (2) anfallenden weiteren Komponenten des Produktstromes einer dritten Kolonne (3) zugeführt werden, in der dritten Kolonne (3) Monomethylamin und Dimethylamin abgetrennt werden und das Monomethylamin und das Dimethylamin in einer vierten Kolonne (4) durch Destillation getrennt werden, **dadurch gekennzeichnet, dass** in den Sumpf (15) oder in den Abtriebsteil der zweiten Kolonne (2) oder dem Zulauf (17) der dritten Kolonne (3) Alkalihydroxid zugegeben wird.

2. Verfahren nach Anspruch 1, **dadurch gekennzeichnet, dass** der dritten Kolonne (3) eine zusätzliche fünfte Kolonne (5) nachgeschaltet ist, die aus einem Seitenabzug oder dem Sumpf der dritten Kolonne (3) gespeist wird und in der Methanol durch Destillation abgetrennt wird.

3. Verfahren nach Anspruch 2, **dadurch gekennzeichnet, dass** das als Sumpf der fünften Kolonne (5) anfallende methanolfreie Wasser in die dritte Kolonne (3) zurückgeführt wird.

4. Verfahren nach einem der Ansprüche 1 bis 3, **dadurch gekennzeichnet, dass** das Alkalihydroxid dem Zulauf (17) der dritten Kolonne (3) zugegeben wird.

5. Verfahren nach einem der Ansprüche 1 bis 3, **dadurch gekennzeichnet, dass** das Alkalihydroxid in den Sumpf (15) oder in den Abtriebsteil der zweiten Kolonne (2) zugegeben wird.

6. Verfahren nach einem der Ansprüche 1 bis 5, **dadurch gekennzeichnet, dass** die Menge des zugegebenen Alkalihydroxid so bemessen ist, dass im Sumpf der dritten Kolonne (3) noch Alkalihydroxid enthalten ist.

7. Verfahren nach einem der Ansprüche 1 bis 6, **dadurch gekennzeichnet, dass** das Alkalihydroxid Natriumhydroxid ist.

8. Verfahren nach einem der Ansprüche 1 bis 6, **dadurch gekennzeichnet, dass** das Alkalihydroxid Kaliumhydroxid ist.

## Claims

1. A method of avoiding corrosion in the separation of methylamines from a product stream (10) which is obtained in the preparation of methylamines by gas-phase reaction of methanol and ammonia and comprises monomethylamine, dimethylamine, trimethylamine, ammonia and methanol as components, where ammonia (11) is separated off by pure distillation in a first column (1), the remaining components of the product stream obtained as bottoms (12) are fed to a second column (2), trimethylamine (14) is separated off in the second column (2) by extractive distillation with introduction of water, the further components of the product stream obtained as bottoms (15) from the second column (2) are fed to a third column (3), monomethylamine and dimethylamine are separated off in the third column (3) and the monomethylamine and dimethylamine are separated by distillation in a fourth column (4), wherein alkali metal hydroxide is added to the bottoms (15) from the second column (2) or introduced into the stripping section of the second column (2) or is added to the feed (17) to the third column (3).

2. The method according to claim 1, wherein an additional fifth column (5) into which a stream taken from a side offtake or the bottom of the third column (3) is fed and in which methanol is separated off by distillation is installed downstream of the third column (3).

3. The method according to claim 2, wherein the methanol-free water obtained as bottoms from the fifth column (5) is recirculated to the third column (3).

4. The method according to any of claims 1 to 3, wherein the alkali metal hydroxide is added to the feed (17) to the third column (3).

5. The method according to any of claims 1 to 3, wherein the alkali metal hydroxide is added to the bottoms (15) from the second column (2) or is introduced into the stripping section of the second column (2).

6. The method according to any of claims 1 to 5, wherein the amount of alkali metal hydroxide added is such that alkali metal hydroxide is still comprised in the bottoms from the third column (3).

7. The method according to any of claims 1 to 6, wherein the alkali metal hydroxide is sodium hydroxide.

8. The method according to any of claims 1 to 6, wherein the alkali metal hydroxide is potassium hydroxide.

## Revendications

1. Procédé pour éviter la corrosion lors de la séparation de méthylamines d'un courant de produit (10) qui se forme lors de la fabrication de méthylamines par une réaction en phase gazeuse de méthanol et d'ammoniac et qui contient de la monométhylamine, de la diméthylamine, de la triméthylamine, de l'ammoniac et du méthanol comme composants, dans lequel, dans une première colonne (1), on sépare par pure distillation de l'ammoniac (11), les composants restants du courant de produit, qui forment le bas (12), sont acheminés à une deuxième colonne (2), on sépare la triméthylamine (14) dans la deuxième colonne (2) par distillation extractive en ajoutant de l'eau, les autres composants du courant de produit, qui forment le bas (15) de la deuxième colonne (2), sont acheminés à une troisième colonne (3), la monométhylamine et la diméthylamine sont séparées dans la troisième colonne (3) et la monométhylamine et la diméthylamine sont séparées dans une quatrième colonne (4) par distillation, **caractérisé en ce que** l'on ajoute de l'hydroxyde de métal alcalin dans le bas (15) ou dans la partie de rectification de la deuxième colonne (2) ou à l'alimentation (17) de la troisième colonne (3).

2. Procédé selon la revendication 1, **caractérisé en ce qu'**en aval de la troisième colonne (3) est montée une cinquième colonne supplémentaire (5), qui est alimentée par soutirage latéral ou par le bas de la troisième colonne (3), et dans laquelle du méthanol est séparé par distillation.

3. Procédé selon la revendication 2, **caractérisé en ce que** l'eau exempte de méthanol, qui se forme dans le bas de la cinquième colonne (5), est recyclée à la troisième colonne (3).

4. Procédé selon l'une quelconque des revendications 1 à 3, **caractérisé en ce que** l'hydroxyde de métal alcalin est ajouté à l'alimentation (17) de la troisième colonne (3).

5. Procédé selon l'une quelconque des revendications 1 à 3, **caractérisé en ce que** l'hydroxyde de métal alcalin est ajouté dans le bas (15) ou dans la partie de rectification de la deuxième colonne (2).

6. Procédé selon l'une quelconque des revendications 1 à 5, **caractérisé en ce que** la quantité d'hydroxyde de métal alcalin ajoutée est mesurée de sorte que de l'hydroxyde de métal alcalin soit encore présent dans le bas de la troisième colonne (3).

7. Procédé selon l'une quelconque des revendications 1 à 6, **caractérisé en ce que** l'hydroxyde de métal alcalin est l'hydroxyde de sodium.

8. Procédé selon l'une quelconque des revendications 1 à 6, **caractérisé en ce que** l'hydroxyde de métal alcalin est l'hydroxyde de potassium.
